# FASCICULE DE BREVET EUROPEEN

(11) **EP 0 997 139 B1**
(45) Date de publication et mention de la délivrance du brevet: **19.09.2001**
(21) Numéro de dépôt: 99401778.8
(22) Date de dépôt: 15.07.1999
(51) Int. Cl.: A61K 7/48

(54) **Composition cosmétique comprenant un polymère filmogène, une poly-alpha-oléfine et une phase grasse liquide**
Kosmetische Zubereitung enthaltend ein filmbildendes Polymer, ein Poly-alpha-olefin und eine flüssige Fettphase
Cosmetic composition containing a film-forming polymer, a poly-alpha-olefin and a liquid fatty phase

(30) Priorité: 04.09.1998 FR 9811101
(43) Date de publication de la demande: 03.05.2000
(73) Titulaire: L'OREAL, 75008 Paris (FR)
(72) Inventeur: Collin, Nathalie, 92330 Sceaux (FR); Yon, Maryline, 75013 Paris (FR); Piot, Bertrand, 75009 Paris (FR)
(74) Mandataire: Lhoste, Catherine

(56) Documents cités:
- EP-A- 0 422 862
- EP-A- 0 819 428
- US-A- 3 937 811

## Description

La présente invention a pour objet une composition filmogène comprenant un polymère filmogène, une huile volatile et une cire de polyoléfine, destinée en particulier aux domaines cosmétique et dermatologique. Plus spécialement, l'invention se rapporte à une composition pour le soin et/ou le maquillage de la peau, des fibres kératiniques (cils, sourcils, cheveux) ou des lèvres d'être humain.

Cette composition peut se présenter notamment sous forme d'eye-liner, de mascara, de produit de maquillage pour le corps, de produit anti-cernes, de fard à paupière ou à joue, de fond de teint, de rouge à lèvres, de composition de protection solaire, de coloration de la peau, de produit de coiffage.

Les produits de maquillage ou de traitement de la peau sont habituellement appliqués sous la forme d'une mince couche uniforme. Pour les compositions destinées au maquillage du bord des paupières, appelés eye-liners, il est souhaitable que le film déposé après l'application soit résistant à l'eau, on dit alors que le produit est waterproof, aux larmes, à la transpiration et au sébum. En outre, il est souhaitable que le film présente une bonne tenue au frottement des doigts.

Il est également souhaitable que le film déposé sur la peau ait des propriétés de non transfert, c'est-à-dire qu'il ne se dépose pas au moins en partie, sans laisser de traces, sur certains supports avec lesquels la peau peut être mise en contact, et notamment un vêtement ou la peau.

Pour un mascara résistant à l'eau, il est aussi souhaitable que le mascara déposé sur les cils présente une bonne tenue, ne transfère pas sur la peau, par exemple lors du contact avec les doigts ou le bord des yeux, et résiste au frottement des doigts et aux larmes.

Pour conférer à ces compositions une bonne tenue sur la peau ou sur les cils, il est connu d'utiliser des polymères filmogènes solubilisés dans un milieu constitué de solvants organiques et éventuellement de cires. Une telle composition est notamment décrite dans le brevet US-A-5480632.

Toutefois, lorsque ces compositions filmogènes sont fluides elles ne présentent pas toujours une bonne homogénéité dans le temps et la consistance de la composition n'est pas uniforme. Cette inhomogénéité est nuisible aux propriétés de maquillage attendues et ne permet pas notamment l'obtention d'un maquillage uniforme. En outre, on observe parfois un épaississement de la composition dans le temps rendant celle-ci difficile à appliquer sur la peau ou sur les cils.

Il est connu du document EP-A-819428 une composition cosmétique sans transfert comprenant un copolymère de vinylpyrrolidone et d'alphaoléfine, un composé volatil. Le brevet US 3937811 décrit une composition cosmétique grasse comprenant une cire et un copolymère d'ester vinylique et d'alphaoléfine.

La présente invention a donc pour but de proposer une composition filmogène présentant une bonne stabilité dans le temps et conduisant à la formation d'un film homogène ayant une bonne tenue et résistant à l'eau.

Les inventeurs ont constaté que l'utilisation d'une cire de polyoléfine particulière dans une composition comprenant une huile volatile et un polymère filmogène permettait d'obtenir une composition parfaitement stable et homogène dans le temps et conduisant à la formation d'un film présentant en particulier une bonne tenue. Le film obtenu est notamment bien résistant à l'eau, au frottement, à la transpiration et au sébum ; il présente également de bonnes propriétés de non transfert.

L'invention a donc pour objet une composition filmogène cosmétique ou dermatologique comprenant une phase grasse liquide, contenant au moins une huile hydrocarbonée volatile, et au moins un polymère filmogène dans la phase grasse liquide, caractérisée par le fait qu'elle comprend au moins une cire de polyoléfine résultant de la polymérisation d'alpha-oléfines ayant au moins 10 atomes de carbone, ladite cire ayant un point de fusion allant de 50 à 80 °C.

L'invention a également pour objet un procédé de maquillage ou de traitement non thérapeutique de la peau et/ou des fibres kératiniques et/ou des lèvres consistant à appliquer la composition selon l'invention sur la peau et/ou les fibres kératiniques et/ou les lèvres.

L'invention a aussi pour objet l'utilisation de la composition selon l'invention pour l'obtention d'un film résistant a l'eau.

L'invention a encore pour objet l'utilisation de la composition selon l'invention pour l'obtention d'un film présentant une bonne tenue aux frottements, et/ou aux larmes, et/ou à la transpiration et/ou au sébum.

L'invention a également pour objet l'utilisation de la composition selon l'invention pour l'obtention d'un film ayant des propriétés de non transfert.

L'invention se rapporte aussi à l'utilisation d'une cire de polyoléfine telle que définie précédemment dans une composition filmogène cosmétique ou dermatologique comprenant une phase grasse liquide, contenant au moins une huile hydrocarbonée volatile, et un polymère filmogène soluble ou dispersible dans ladite phase grasse liquide, pour l'obtention d'une composition homogène.

### a) la cire de polyoléfine :

La cire de polyoléfine utilisée dans la composition selon l'invention est issue d'homopolymérisation, d'alpha-oléfine répondant à la formule générale : R-CH=CH₂ dans laquelle R désigne un radical alkyle ayant de 10 à 50 atomes de carbone, et de préférence de 25 à 50 atomes de carbone. De préférence, R est un radical alkyl linéaire.

Selon l'invention, on entend par homopolymérisation d'alpha-oléfine la polymérisation de monomères consistant essentiellement en une alpha-oléfine ou un mélange d'alpha-oléfine telles que définies précédemment.

La cire de polyoléfine a avantageusement une pénétrabilité à l'aiguille, mesurée à 44 °C, allant de 15 à 120, de préférence de 100 à 120, et mieux de 105 à 115. La cire de polyoléfine a de préférence un point de fusion allant de 50 °C à 60 °C. La cire de polyoléfine peut avoir un poids moléculaire moyen en nombre allant de 400 à 3000, de préférence de 2000 à 3000 et mieux de 2500 à 2700.

De telles cires de polyoléfine sont décrites dans les brevets US-A-4060569 et USA-4239546. Ces cires sont notamment vendues sous la dénomination de "PERFORMA V® 103", "PERFORMA V® 253", "PERFORMA V® 260" par la société PETROLITE.

Ces cires présentent les caractéristiques suivantes :

| | PERFORMA V® 103 | PERFORMA V® 253 | PERFORMA V® 260 |
|---|---|---|---|
| point de fusion (norme ASTM D 36) | 74 °C | 67 °C | 54°C |
| poids moléculaire moyen en nombre : | 2800 | 520 | 2600 |
| polydispersité du poids moléculaire | 6 | 8 | 11,5 |
| densité mesurée à 25 °C (norme ASTM D 792) | 0,92 g/cm ³ | 0,92 g/cm ³ | 0,90 g/cm ³ |
| dureté (pénétrabilité à l'aiguille - norme ASTM D 1321) à 25 °C | 5 | 7 | 12 |
| dureté (pénétrabilité à l'aiguille - norme ASTM D 1321) à 44 °C | 20 | - | 110 |
| viscosité à 99°C (norme ASTM D 792) | 0,345 Pa.s (345 centipoises) | 0,006 Pa.s (6 centipoises) | 0,358 Pa.s. (358 centipoises) |

La polydispersité du poids moléculaire correspond au rapport du poids moléculaire moyen en poids sur le poids moléculaire moyen en nombre.

La pénétrabilité à l'aiguille des cires est déterminée selon la norme française NF T 60-123 ou la norme américaine ASTM D 1321, à la température de 44 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

La cire de polyoléfine a de préférence une densité allant de 0,85 à 0,95 g/cm³.

La cire de polyoléfine peut être présente, dans la composition selon l'invention, en une teneur allant de 0,1 % à 10 % en poids, par rapport au poids total de la composition, et de préférence de 1 % à 5 % en poids.

### b) la phase grasse liquide :

On entend par "phase grasse liquide" dans la présente invention, tout milieu non aqueux liquide à température ambiante. On entend par "huile volatile" une huile susceptible de s'évaporer à température ambiante d'un support sur lequel elle a été appliquée, autrement dit une huile ayant une tension de vapeur mesurable à température ambiante. On entend par "huile hydrocarbonée", une huile ne contenant que des atomes d'hydrogène et de carbone.

Les huiles hydrocarbonées volatiles préférées convenant pour la composition selon l'invention sont en particulier les isoparaffines, à savoir des alcanes ramifiés, comportant de 8 à 16 atomes de carbone telles que les 'ISOPARs', les PERMETYLs et notamment l'isododécane (encore appelé 2,2,4,4,6-pentaméthylheptane). On peut bien entendu également utiliser des mélanges de telles isoparaffines. D'autres huiles hydrocarbonées volatiles comme les distillats de pétrole, notamment ceux vendus sous la dénomination Shell Solt par la société SHELL, peuvent aussi être utilisés.

L'huile hydrocarbonée volatile peut être présente dans la composition selon l'invention en une teneur allant de 20 % à 99,4 % en poids, par rapport au poids total de la composition, de préférence de 40 % à 80 % en poids, et mieux de 50 % à 70 % en poids.

La composition selon l'invention peut également contenir un ou plusieurs solvants volatils supplémentaires, différents des huiles hydrocarbonées volatiles de la présente invention. On peut citer à titre d'exemple de ces solvants volatils supplémentaires les huiles de silicones cycliques et volatiles telles que l'octaméthylcyclotétrasiloxane, le décaméthylcyclopentasiloxane, l'hexadécaméthylcyclohexasiloxane, les silicones linéaires volatiles telles que l'octaméthyltrisiloxane, l'heptaméthylhexyltrisiloxane, l'heptaméthyloctyltrisiloxane, et les huiles volatiles fluorées telles que le nonafluorométhoxybutane ou le perfluorométhylcyclopentane. Ces solvants volatils représentent de préférence de 0 à 15 % en poids de la phase volatile (notamment 0,1 % à 15 %).

La composition selon l'invention peut également contenir des huiles non volatiles, et notamment des huiles hydrocarbonées et/ou siliconées et/ou fluorées non volatiles.
Comme huile hydrocarbonée non volatile, on peut notament citer :
- les huiles hydrocarbonées d'origine animale telle que le perhydrosqualène;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité;
- les hydrocarbures linéaires ou ramifiés, d'origine minérale ou synthétique tels que la vaseline, les polydécènes, le polyisobutène hydrogéné tel que le parléam;
- les esters et les éthers de synthèse comme les huiles de formule R₁₀COOR₁₁ dans laquelle R₁₀ représente le reste d'un acide gras supérieur comportant de 6 à 29 atomes de carbone et R₁₁ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyldodécyle ; les esters de polyols comme le dioctanoate de propylène glycol, le diheptanoate de néopentylglycol, le diisonanoate de diéthylène glycol et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol;
- les acides gras supérieurs tels que l'acide myristique, l'acide palmitique, l'acide stéarique, l'acide béhénique, l'acide oléique, l'acide linoléique, l'acide linolénique ou l'acide isostéarique ;
et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles non volatiles peuvent être présentes dans la composition selon l'invention en une teneur allant de 0 % à 5 % en poids (notamment 0,1 % à 5 %), par rapport au poids total de la composition, de préférence de 0 % à 2 % en poids, et mieux de 0,1 % à 2 % en poids.

### c) le polymère filmogène :

Le polymère filmogène utilisable dans la composition de la présente demande est en particulier soluble, ou dit encore liposoluble, dans la phase grasse liquide de la composition. Il peut être présent dans la composition en une teneur allant de 0,5 % à 20 % en poids, par rapport au poids total de la composition, et de préférence de 1 % à 15 % en poids. Ce polymère filmogène confère notamment une bonne tenue de la composition après application sur la peau, les fibres kératiniques ou les lèvres.

On entend par "polymère filmogène" un polymère capable de former seul un film isolable.

A titre d'exemple de polymère liposoluble, on peut citer les polymères correspondant à la formule (I) suivante : 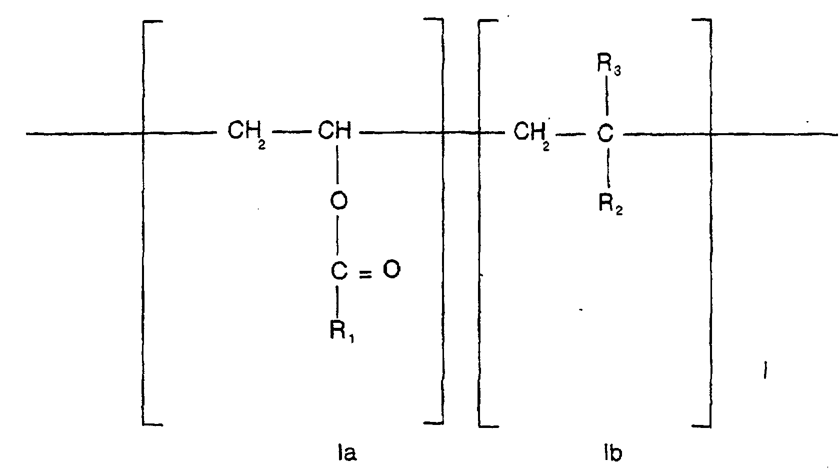 dans laquelle :
- R₁ représente une chaîne hydrocarbonée saturée, linéaire ou ramifiée, ayant de 1 à 19 atomes de carbone ;
- R₂ représente un radical pris dans le groupe constitué par :
   a) -O-CO-R₄, R₄ ayant la même signification que R₁ mais est différent de R₁ dans un même copolymère,
   b) -CH₂-R₅, R₅ représentant une chaîne hydrocarbonée saturée, linéaire ou ramifiée, ayant de 5 à 25 atomes de carbone,
   c) -O-R₆, R₆ représentant une chaîne hydrocarbonée saturée, ayant de 2 à 18 atomes de carbone, et
   d) -CH₂-O-CO-R₇, R₇ représentant une chaîne hydrocarbonée saturée, linéaire ou ramifiée, ayant de 1 à 19 atomes de carbone,
- R₃ représente un atome d'hydrogène quand R₂ représente les radicaux a), b) ou c) ou R₃ représente un radical méthyle quand R₂ représente le radical d), ledit copolymère devant être constitué d'au moins 15 % en poids d'au moins un monomère dérivé d'un motif (la) ou d'un motif (lb) dans lesquels les chaînes hydrocarbonées, saturées ou ramifiées, ont au moins 7 atomes de carbone.

Les copolymères de formule (I) résultent de la copolymérisation d'au moins un ester vinylique (correspondant au motif la) et d'au moins un autre monomère (correspondant au motif lb) qui peut être une α-oléfine, un alkylvinyléther ou un ester allylique ou méthalylique.

Lorsque dans le motif (lb) R₂ est choisi parmi les radicaux -CH₂-R₅, -O-R₆ ou -CH₂-O-CO-R₇ tels que définis précédemment, le copolymère de formule (I) peut être constitué de 50 à 95 % en moles d'au moins un motif (la) et de 5 à 50 % en moles d'au moins un motif (lb).

Les copolymères de formule (I) peuvent également résulter de la copolymérisation d'au moins un ester vinylique et d'au moins un autre ester vinylique différent du premier. Dans ce cas, ces copolymères peuvent être constitués de 10 à 90 % en moles d'au moins un motif (la) et de 10 à 90% en moles d'au moins un motif (lb) dans lequel R₂ représente le radical -O-CO-R₄.

Parmi les esters vinyliques conduisant au motif de formule (la), ou au motif de formule (lb) dans lequel R₂ = -O-CO-R₄, on peut citer l'acétate de vinyle, le propionate de vinyle, le butanoate de vinyle, l'octanoate de vinyle, le décanoate de vinyle, le laurate de vinyle, le stéarate de vinyle, l'isostéarate de vinyle, le diméthyl-2, 2 octanoate de vinyle, et le diméthylpropionate de vinyle.

Parmi les α-oléfines conduisant au motif de formule (lb) dans lequel R₂ = -CH₂-R₅, on peut citer l'octène-1, le dodécène-1, l'octadécène-1, l'eicosène-1, et les mélanges d'α-oléfines ayant de 22 à 28 atomes de carbone.

Parmi les alkylvinyléthers conduisant au motif de formule (lb) dans lequel R₂ = -O-R₆, on peut citer l'éthylvinyléther, le n-butylvinyléther, l'isobutylvinyléther, le décylvinyléther, le dodécylvinyléther, le cétylvinyléther et l'octadécylvinyléther.

Parmi les esters allyliques ou méthallyliques conduisant au motif de formule (lb) dans lequel R₂ = -CH₂-O-CO-R₇, on peut citer les acétates, les propionates, les diméthylpropionates, les butyrates, les hexanoates, les octanoates, les décanoates, les laurates, les diméthyl-2, 2 pentanoates, les stéarates et les eicosanoates d'allyle et de méthallyle.

Les copolymères de formule (I) peuvent également être réticulés à l'aide de certains types de réticulants qui ont pour but d'augmenter sensiblement leur poids moléculaire.

Cette réticulation est effectuée lors de la copolymérisation et les réticulants peuvent être soit du type vinylique, soit du type allylique ou méthallylique. Parmi ceux-ci, on peut citer en particulier le tétraallyloxyéthane, le divinylbenzène, l'octanedioate de divinyle, le dodécanedioate de divinyle, et l'octadécanedioate de divinyle.

Parmi les différents copolymères de formule (I) utilisables dans la composition selon l'invention, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2262303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, différents de la cire de polyoléfine définie en a), comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

### d) les additifs :

La phase grasse de la composition selon l'invention peut contenir, outre les huiles hydrocarbonées volatiles et la cire de polyoléfine définies précédemment, une ou plusieurs cires d'origine animale, végétale ou synthétique.

Les cires additionnelles susceptibles d'être utilisées dans la composition selon l'invention possèdent en règle générale un point de fusion compris entre 40 et 110 °C et ont une pénétration à l'aiguille, à 25 °C, comprise entre 3 et 40, telle que mesurée selon la norme américaine ASTM D 5 ou selon la norme française NFT 004. Le principe de la mesure de pénétration d'une aiguille selon les normes ASTM D 5 et NFT 004 consiste à mesurer la profondeur exprimée en dixième de millimètres, à laquelle pénètre une aiguille normalisée qui pèse 2,5 g placée dans un porte-aiguilles pesant 47,5 g soit un total de 50 g, l'aiguille étant placée sur la cire pendant 5 secondes.
Parmi les cires d'origine animale, on peut citer les cires d'abeille, les cires de lanoline et les cires d'insectes de Chine.
Parmi les cires d'origine végétale, on peut citer les cires de riz, les cires de Carnauba, de Candellila, d'Ouricurry, les cires de fibres de liège, les cires de canne à sucre, les cires du Japon, la cire de Sumac, la cire de coton.
Parmi les cires d'origine minérale, on peut citer les paraffines, les cires microcristallines, les cires de Montan et les ozokérites.
Parmi les cires d'origine synthétique, on peut utiliser notamment les cires de polyéthylène, les cires obtenues par la synthèse de Fischer et Tropsch, les copolymères cireux ainsi que leurs esters, les cires de silicone.
Il est également possible d'utiliser des huiles d'origine animales ou végétales hydrogénées qui répondent toujours aux deux caractéristiques physiques mentionnées précédemment.
Parmi ces huiles, on peut citer les cires de jojoba hydrogénées et les huiles hydrogénées qui sont obtenues par hydrogénation catalytique de corps gras composés de chaîne grasse linéaire ou non en C₈-C₃₂, l'huile de tournesol hydrogénée, l'huile de ricin hydrogénée, l'huile de coprah hydrogénée, la lanoline hydrogénée et les huiles de palme hydrogénées. Les cires utilisables selon la présente invention sont de préférence solides et rigides à température inférieure à 50 °C.

Avantageusement, la composition selon l'invention peut comprendre de 0 % à 30 % (notamment 0,1 % à 30 %) en poids de cire, en poids par rapport au poids total de la composition, de préférence de 1 % à 20 % en poids, et mieux de 1 % à 10 % en poids.

La composition selon l'invention peut comprendre, en outre, des gommes de silicones. Les gommes de silicone peuvent être des polysiloxanes de masse moléculaire élevée, de l'ordre de 200 000 à 1 000 000 et ayant une viscosité supérieure à 500 000 mPa.s. Elles peuvent être utilisées seules ou en mélange avec un solvant tel qu'une huile polydiméthylsiloxane ou polyphénylsiloxane. Les gommes peuvent être présentes dans la composition en une teneur allant de 0 % à 2 % (notamment 0,1 % à 2 %) en poids, par rapport au poids total de la composition, et de préférence de 0,1 % à 1 % en poids.

La composition selon l'invention peut comprendre, en outre, un agent épaississant de la phase grasse liquide. L'agent épaississant peut être choisi parmi les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX.

L'agent épaississant peut être présent en une teneur allant de 0,5 % à 10 % en poids, par rapport au poids total de la composition, et mieux de 1 % à 6 % en poids.

La composition peut également comprendre au moins une matière colorante comme les composés pulvérulents et/ou les colorants liposolubles, par exemple à raison de 0,01 à 30 % du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques.

La composition peut comprendre, en outre, tout additif habituellement utilisés dans de telles compositions, tels que les conservateurs, les parfums, les filtres solaires, les agents anti-radicaux libres, les agents hydratants, les vitamines, les protéines, les céramides, les plastifiants.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention est avantageusement anhydre, et peut contenir moins de 10 % en poids d'eau par rapport au poids total de la composition. La phase aqueuse éventuellement présente dans la composition peut contenir un additif cosmétiquement ou dermatologiquement acceptable.

La composition selon l'invention peut se présenter sous la forme fluide, gélifiée, semi-solide, pâte souple, voire solide telle que stick ou bâton. Ces formes galéniques sont préparées selon les méthodes usuelles des domaines considérés.

La composition selon l'invention peut être utilisée pour le maquillage ou le traitement cosmétique de la peau, des fibres kératiniques ou des lèvres. La composition de maquillage peut être un eye-liner, un mascara, un fond de teint, un fard à paupières ou a joue, un rouge à lèvres, un produit anti-cernes, ou encore un produit de maquillage du corps du type tatouage provisoire ou semi-permanent. La composition de traitement cosmétique peut être une composition de soin pour le visage, le cou, les mains ou pour le corps ; elle peut constituer aussi une composition solaire ou autobronzante.

L'invention est illustrée plus en détail dans les exemples suivants.

### Exemple 1 :

On a préparé un eye-liner ayant la composition suivante :
- Cires 4,7 %
- Pigments 11 %
- Agent épaississant 7 %
- Cire de polyoléfine (PERFORMA V® 260 de PETROLITE) 3,7 %
- Copolymère acétate de vinyle/Stéarate d'allyle (65/35) (polymère liposoluble) 7,5 %
- Charge 1 %
- Hydrocarbures paraffiniques et naphténiques légers (Shell Solt de SHELL) 28,5 %
- Hydrocarbures iso-paraffiniques (Isopar E d'ESSO) 36,6 %

On obtient un eye-liner qui s'applique facilement sur le bord des paupières et laisse, après l'application, un film homogène présentant une bonne tenue dans le temps à l'eau et à la transpiration. Le film ne s'altère pas pendant la journée.

### Exemple 2 :

On a préparé un produit pour le maquillage du corps ayant la composition suivante :
- Cire de polyoléfine (PERFORMA V® 260 de PETROLITE) 3,7 %
- Copolymère vinylpyrrolidone/triacontène (ANTARON WP-660 de GAF) 4 %
- Copolymère vinylpyrrolidone/eicosène (ANTARON V-220 de GAF) 4 %
- Agent épaississant 7 %
- Charge 1 %
- Pigments 11 %
- Hydrocarbures paraffiniques et naphténiques légers (Shell Soit de SHELL) 30 %
- Hydrocarbures iso-paraffiniques (Isopar E d'ESSO) 39,3 %

La composition s'applique facilement sur la peau et laisse un film ayant de bonne propriété de sans transfert et résistant aux frottements des vêtement et de la peau.

### Exemple 3 :

On a préparé un mascara ayant la composition suivante :
- Cire de polyéthylène 8 %
- Cire de polyoléfine (PERFORMA V® 260 de PETROLITE) 1 %
- Copolymère vinylpyrrolidone/triacontène (ANTARON WP-660 de GAF) 3,5 %
- Copolymère vinylpyrrolidone/eicosène (ANTARON V-220 de GAF) 3,5 %
- Agent épaississant 7 %
- Charge 1 %
- Pigments 11 %
- Hydrocarbures paraffiniques et naphténiques légers (Shell Solt de SHELL) 28,5 %
- Hydrocarbures iso-paraffiniques (Isopar E d'ESSO) 36,5 %

Le mascara s'applique facilement sur les cils et présente une bonne tenue tout au long de la journée. Le maquillage obtenu est homogène et résistant à l'eau.

## Revendications

1. Composition filmogène cosmétique ou dermatologique comprenant une phase grasse liquide, contenant au moins une huile hydrocarbonée volatile, et un polymère filmogène dans ladite phase grasse liquide, **caractérisée par** le fait qu'elle comprend au moins une cire de polyoléfine résultant de l'homopolymérisation d'alpha-oléfine répondant à la formule générale : R-CH=CH₂ dans laquelle R désigne un radical alkyle ayant de 10 à 50 atomes de carbone, ladite cire ayant un point de fusion allant de 50 °C à 80 °C.

2. Composition selon la revendication 1, **caractérisée par** le fait que la cire de polyoléfine a un point de fusion allant de 50 °C à 60 °C.

3. Composition selon la revendication 1 ou 2, **caractérisée par** le fait que la cire de polyoléfine a une pénétrabilité à l'aiguille, mesurée à 44 °C, allant de 15 à 120.

4. Composition selon l'une quelconque des revendications précédentes, **caractérisée par** le fait que la cire de polyoléfine a une pénétrabilité à l'aiguille, mesurée à 44 °C, allant de 100 à 120, et mieux de 105 à 115.

5. Composition selon l'une quelconque des revendications précédentes, **caractérisée par** le fait que ladite cire de polyoléfine a un poids moléculaire moyen en nombre allant de 400 à 3000.

6. Composition selon l'une quelconque des revendications précédentes, **caractérisée par** le fait que ladite cire de polyoléfine à un poids moléculaire moyen en nombre allant de 2000 à 3000, et mieux de 2500 à 2700.

7. Composition selon l'une quelconque des revendications précédentes, **caractérisée par** le fait que ladite cire de polyoléfine a une densité allant de 0,85 à 0,95 g/cm³.

8. Composition selon la revendication 1, **caractérisée par** le fait que R désigne un radical alkyle ayant de 25 à 50 atomes de carbone.

9. Composition selon l'une des revendications 1 ou 8, **caractérisée par** le fait que le radical alkyl R est linéaire.

10. Composition selon l'une quelconque des revendications précédentes, **caractérisée par** le fait que la cire de polyoléfine est présente en une teneur allant de 0,1 % à 10 % en poids, par rapport au poids total de la composition.

11. Composition selon l'une quelconque des revendications précédentes, **caractérisée par** le fait que la cire de polyoléfine est présente en une teneur allant de 1 % à 5 % en poids, par rapport au poids total de la composition.

12. Composition selon l'une quelconque des revendications précédentes, **caractérisée par** le fait que l'huile hydrocarbonée volatile est choisie dans le groupe formé par les isoparaffines comportant de 8 à 16 atomes de carbone.

13. Composition selon l'une quelconque des revendications précédentes, **caractérisée par** le fait que l'huile hydrocarbonée volatile est présente en une teneur allant de 20 % à 99,4 % en poids, par rapport au poids total de la composition.

14. Composition cosmétique selon l'une quelconque des revendications précédentes, **caractérisée par** le fait que l'huile hydrocarbonée volatile est présente en une teneur allant de 40 % à 80 % en poids, par rapport au poids total de la composition, et mieux de 50 % à 70 % en poids.

15. Composition selon l'une quelconque des revendications précédentes, **caractérisée par** le fait que la phase grasse liquide comprend en outre un ou plusieurs solvants volatils différents des huiles hydrocarbonée volatiles.

16. Composition selon l'une quelconque des revendications précédentes, **caractérisée par** le fait que la phase grasse liquide comprend en outre au moins une huile non volatile d'origine minérale, animale, végétale ou synthétique.

17. Composition selon l'une quelconque des revendications précédentes, **caractérisée par** le fait que le polymère filmogène est soluble dans la phase grasse liquide.

18. Composition selon la revendication 17, **caractérisée par** le fait que le polymère filmogène est choisi parmi les copolymères de formule (I): 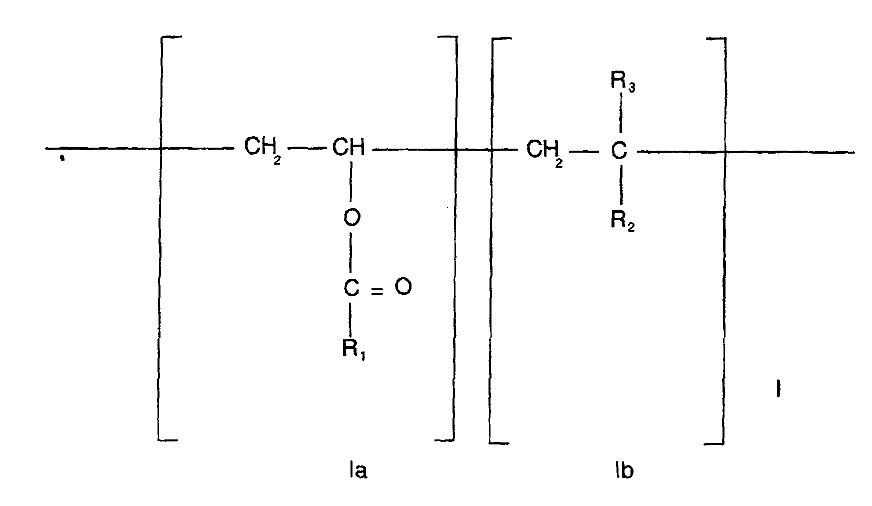 dans laquelle
- R₁ représente une chaîne hydrocarbonée saturée, linéaire ou ramifiée, ayant de 1 à 19 atomes de carbone ;
- R₂ représente un radical pris dans le groupe constitué par :
a) -O-CO-R₄, R₄ ayant la même signification que R₁ mais est différent de R₁ dans un même copolymère,
b) -CH₂-R₅, R₅ représentant une chaîne hydrocarbonée saturée, linéaire ou ramifiée, ayant de 5 à 25 atomes de carbone,
c) -O-R₆, R₆ représentant une chaîne hydrocarbonée saturée, ayant de 2 à 18 atomes de carbone, et
d) -CH₂-O-CO-R₇, R₇ représentant une chaîne hydrocarbonée saturée, linéaire ou ramifiée, ayant de 1 à 19 atomes de carbone,
- R₃ représente un atome d'hydrogène quand R₂ représente les radicaux a), b) ou c) ou R₃ représente un radical méthyle quand R₂ représente le radical d), ledit copolymère devant être constitué d'au moins 15 % en poids d'au moins un monomère dérivé d'un motif (la) ou d'un motif (lb) dans lesquels les chaînes hydrocarbonées, saturées ou ramifiées, ont au moins 7 atomes de carbone.

19. Composition selon l'une des revendications 17 ou 18, **caractérisée par** le fait que le polymère filmogène est un homoplymère choisi dans le groupe formé par les homopolymères résultant de l'homopolymérisation d'esters vinyliques ayant de 9 à 22 atomes de carbone ou (méth)acrylates d'alkyle dont le radical alkyle a de 10 à 20 atomes de carbone.

20. Composition selon l'une quelconque des revendications 17 à 19, **caractérisée par** le fait que le polymère filmogène est choisi dans le groupe formé par les copolymères d'alcènes en C₂-C₂₀, les alkylcelluloses avec un radical alkyl linéaire ou ramifié, saturé ou non en C₁ à C₈, les copolymères de la vinylpyrolidone.

21. Composition selon l'une quelconque des revendications 17 à 20, **caractérisée par** le fait que le polymère filmogène est choisi parmi les copolymères de la vinylpyrrolidone et d'alcène en C₂ à C₄₀.

22. Composition selon l'une quelconque des revendications précédentes, **caractérisée par** le fait que le polymère filmogène est présent en une teneur allant de 0,5 % à 20 % en poids, par rapport au poids total de la composition, et de préférence de 1 % à 15 % en poids.

23. Composition selon l'une quelconque des revendications précédentes, **caractérisée par** le fait qu'elle comprend en outre une ou plusieurs cires additionnelles d'origine animale, végétale ou synthétique.

24. Composition selon la revendication 23, **caractérisée par** le fait que les cires d'origine animale, végétale ou synthétique différents de ladite cire de polyoléfine sont présent en une teneur allant de 0 % à 30 % en poids, par rapport au poids total de la composition, et de préférence de 1 à 20 % en poids.

25. Composition selon l'une quelconque des revendications précédentes, **caractérisée par** le fait qu'elle comprend en outre une gomme de silicone.

26. Composition selon l'une quelconque des revendications précédentes, **caractérisée par** le fait qu'elle comprend au moins une matière colorante choisie dans le groupe formé par les composés pulvérulents et les colorants liposolubles.

27. Composition selon la revendication 26, **caractérisée par** le fait que les composés pulvérulents sont choisis dans le groupe formé par les pigments, les nacres et les charges.

28. Composition cosmétique selon l'une quelconque des revendications précédentes, **caractérisée par** le fait qu'elle comprend au moins un agent épaississant de la phase grasse liquide.

29. Composition selon l'une quelconque des revendications précédentes, **caractérisée par** le fait qu'elle comprend au moins un additif choisi dans le groupe formé par les conservateurs, les parfums, les filtres solaires, les agents anti-radicaux libres, les agents hydratants, les vitamines, les protéines, les céramides, les plastifiants.

30. Composition selon l'une quelconque des revendications précédentes, **caractérisée par** le fait que la composition est anhydre.

31. Composition selon l'une quelconque des revendications précédentes, **caractérisée par** le fait que la composition se présente sous la forme de composition de maquillage ou de traitement cosmétique de la peau, des fibres kératiniques ou des lèvres.

32. Procédé de maquillage de la peau et/ou des fibres kératiniques et/ou des lèvres, **caractérisé par** le fait que l'on applique sur la peau et/ou les fibres kératiniques et/ou les lèvres une composition selon l'une quelconque des revendications 1 à 31.

33. Procédé de traitement non thérapeutique pour le soin de la peau et/ou des fibres kératiniques et/ou des lèvres, **caractérisé par** le fait que l'on applique sur la peau et/ou les fibres kératiniques et/ou les lèvres une composition selon l'une quelconque des revendications 1 à 31.

34. Utilisation cosmétique d'une composition telle que définie selon l'une quelconque des revendications 1 à 31 pour l'obtention d'un film ayant des propriétés de non transfert.

35. Utilisation cosmétique d'une composition telle que définie selon l'une quelconque des revendications 1 à 31 pour l'obtention d'un film présentant une bonne tenue aux frottements, et/ou aux larmes, et/ou à la transpiration et/ou au sébum.

36. Utilisation cosmétique d'une composition telle que définie selon l'une quelconque des revendications 1 à 31 pour l'obtention d'un film résistant a l'eau.

## Patentansprüche

1. Filmbildende kosmetische oder dermatologische Zusammensetzung, die eine flüssige Fettphase mit mindestens einem flüchtigen Kohlenwasserstofföl und in der flüssigen Fettphase ein filmbildendes Polymer enthält,
**dadurch gekennzeichnet, daß**
sie mindestens ein Polyolefinwachs enthält, das bei der Homopolymerisation eines α-Olefins mit der folgenden allgemeinen Formel entsteht: R-CH=CH₂, worin R eine Alkylgruppe mit 10 bis 50 Kohlenstoffatomen bedeutet, wobei das Wachs einen Schmelzpunkt von 50 bis 80 °C aufweist.

2. Zusammensetzung nach Anspruch 1, **dadurch gekennzeichnet, daß** das Polyolefinwachs einen Schmelzpunkt von 50 bis 60 °C aufweist.

3. Zusammensetzung nach Anspruch 1 oder 2, **dadurch gekennzeichnet, daß** das Polyolefinwachs eine bei 44 °C bestimmte Nadelpenetration von 15 bis 120 aufweist.

4. Zusammensetzung nach einem der vorhergehenden Ansprüche,
**dadurch gekennzeichnet, daß** das Polyolefinwachs eine bei 44 °C bestimmte Nadelpenetration von 100 bis 120 und besser 105 bis 115 aufweist.

5. Zusammensetzung nach einem der vorhergehenden Ansprüche,
**dadurch gekennzeichnet, daß** das Polyolefinwachs ein Zahlenmittel des Molekulargewichts von 400 bis 3000 aufweist.

6. Zusammensetzung nach einem der vorhergehenden Ansprüche,
**dadurch gekennzeichnet, daß** das Polyolefinwachs ein Zahlenmittel des Molekulargewichts von 2000 bis 3000 und besser 2500 bis 2700 aufweist.

7. Zusammensetzung nach einem der vorhergehenden Ansprüche,
**dadurch gekennzeichnet, daß** das Polyolefinwachs eine Dichte von 0,85 bis 0,95 g/cm³ aufweist.

8. Zusammensetzung nach Anspruch 1, **dadurch gekennzeichnet, daß** R eine Alkylgruppe von 25 bis 50 Kohlenstoffatomen bedeutet.

9. Zusammensetzung nach einem der Ansprüche 1 oder 8, **dadurch gekennzeichnet, daß** die Alkylgruppe geradkettig ist.

10. Zusammensetzung nach einem der vorhergehenden Ansprüche,
**dadurch gekennzeichnet, daß** das Polyolefinwachs in einer Menge von 0,1 bis 10 Gew.-%, bezogen auf das Gesamtgewicht der Zusammensetzung, vorliegt.

11. Zusammensetzung nach einem der vorhergehenden Ansprüche,
**dadurch gekennzeichnet, daß** das Polyolefinwachs in einer Menge von 1 bis 5 Gew.-%, bezogen auf das Gesamtgewicht der Zusammensetzung, vorliegt.

12. Zusammensetzung nach einem der vorhergehenden Ansprüche,
**dadurch gekennzeichnet, daß** das flüchtige Kohlenwasserstofföl unter den Isoparaffinen mit 8 bis 16 Kohlenstoffatomen ausgewählt ist.

13. Zusammensetzung nach einem der vorhergehenden Ansprüche, **dadurch gekennzeichnet, daß** das flüchtige Kohlenwasserstofföl in einer Menge von 20 bis 99,4 Gew.-%, bezogen auf das Gesamtgewicht der Zusammensetzung, vorliegt.

14. Kosmetische Zusammensetzung nach einem der vorhergehenden Ansprüche, **dadurch gekennzeichnet, daß** das flüchtige Kohlenwasserstofföl in einer Menge von 40 bis 80 Gew.-%, bezogen auf das Gesamtgewicht der Zusammensetzung, und besser 50 bis 70 Gew.-% vorliegt.

15. Zusammensetzung nach einem der vorhergehenden Ansprüche,
**dadurch gekennzeichnet, daß** die flüssige Fettphase ferner ein oder mehrere flüchtige Lösungsmittel enthält, die von den flüchtigen Kohlenwasserstoffölen verschieden sind.

16. Zusammensetzung nach einem der vorhergehenden Ansprüche,
**dadurch gekennzeichnet, daß** die flüssige Fettphase ferner mindestens ein nicht flüchtiges Öl mineralischer, tierischer, pflanzlicher oder synthetischer Herkunft enthält.

17. Zusammensetzung nach einem der vorhergehenden Ansprüche,
**dadurch gekennzeichnet, daß** das filmbildende Polymer in der flüssigen Fettphase löslich ist.

18. Zusammensetzung nach Anspruch 17, **dadurch gekennzeichnet, daß** das filmbildende Polymer unter den Copolymeren der Formel (I) ausgewählt ist: 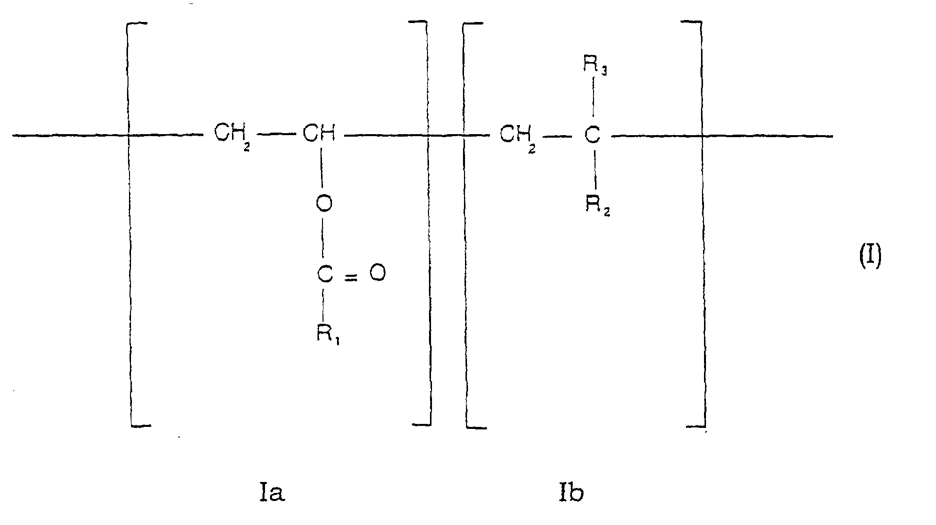 worin bedeuten:
- R₁ eine gesättigte, geradkettige oder verzweigte Kohlenwasserstoffkette mit 1 bis 19 Kohlenstoffatomen;
- R₂ eine Gruppe, die ausgewählt ist unter:
a) -O-CO-R₄, wobei R₄ die Bedeutung von R₁ aufweist, jedoch in dem gleichen Copolymer von R₁ verschieden ist;
b) -CH₂-R₅, wobei R₅ eine gesättigte, geradkettige oder verzweigte Kohlenwasserstoffkette mit 5 bis 25 Kohlenstoffatomen bedeutet;
c) -O-R₆, wobei R₆ eine gesättigte Kohlenwasserstoffkette mit 2 bis 18 Kohlenstoffatomen bedeutet, und
d) -CH₂-O-CO-R₇, wobei R₇ eine gesättigte, geradkettige oder verzweigte Kohlenwasserstoffkette mit 1 bis 19 Kohlenstoffatomen bedeutet;
- R₃ ein Wasserstoffatom, wenn R₂ die Gruppe a), b) oder c) bedeutet oder eine Methylgruppe, wenn R₂ die Gruppe d) bedeutet,
wobei das Copolymer aus mindestens 15 Gew.-% mindestens eines Monomers bestehen muß, das von einer Einheit (Ia) oder einer Einheit (Ib) abgeleitet ist, worin die gesättigten oder verzweigten Kohlenwasserstoffketten mindestens 7 Kohlenstoffatome aufweisen.

19. Zusammensetzung nach einem der Ansprüche 17 oder 18, **dadurch gekennzeichnet, daß** das filmbildende Polymer ein Homopolymer ist, das unter den Homopolymeren ausgewählt ist, die bei der Homopolymerisation von Vinylestern mit 9 bis 22 Kohlenstoffatomen oder Alkyl(meth)acrylaten entstehen, deren Alkylgruppe 10 bis 20 Kohlenstoffatome aufweist.

20. Zusammensetzung nach einem der Ansprüche 17 bis 19, **dadurch gekennzeichnet, daß** das filmbildende Polymer unter den Copolymeren von C₂₋₂₀-Alkenen, Alkylcellulosen mit geradkettiger oder verzweigter, gesättigter oder ungesättigter C₁₋₈-Alkylgruppe und Vinylpyrrolidoncopolymeren ausgewählt ist.

21. Zusammensetzung nach einem der Ansprüche 17 bis 20, **dadurch gekennzeichnet, daß** das filmbildende Polymer unter den Copolymeren von Vinylpyrrolidon und einem C₂₋₄₀-Alken ausgewählt ist.

22. Zusammensetzung nach einem der vorhergehenden Ansprüche, **dadurch gekennzeichnet, daß** das filmbildende Polymer in einer Menge von 0,5 bis 20 Gew.-%, bezogen auf das Gesamtgewicht der Zusammensetzung, und vorzugsweise 1 bis 15 Gew.-% vorliegt.

23. Zusammensetzung nach einem der vorhergehenden Ansprüche, **dadurch gekennzeichnet, daß** sie außerdem ein oder mehrere zusätzliche Wachse tierischer, pflanzlicher oder synthetischer Herkunft enthält.

24. Zusammensetzung nach Anspruch 23, **dadurch gekennzeichnet, daß** die Wachse tierischer, pflanzlicher oder synthetischer Herkunft, die von dem Polyolefinwachs verschieden sind, in einer Menge von 0 bis 30 Gew.-%, bezogen auf das Gesamtgewicht der Zusammensetzung, und vorzugsweise 1 bis 20 Gew.-% vorliegen.

25. Zusammensetzung nach einem der vorhergehenden Ansprüche, **dadurch gekennzeichnet, daß** sie ferner einen Silicongummi enthält.

26. Zusammensetzung nach einem der vorhergehenden Ansprüche, **dadurch gekennzeichnet, daß** sie mindestens ein Farbmittel enthält, das unter den pulverförmigen Verbindungen und fettlöslichen Farbmitteln ausgewählt ist.

27. Zusammensetzung nach Anspruch 26, **dadurch gekennzeichnet, daß** die pulverförmigen Verbindungen unter den Pigmenten, Perlglanzpigmenten und Füllstoffen ausgewählt sind.

28. Kosmetische Zusammensetzung nach einem der vorhergehenden Ansprüche, **dadurch gekennzeichnet, daß** sie mindestens ein Verdickungsmittel für die flüssige Fettphase enthält.

29. Zusammensetzung nach einem der vorhergehenden Ansprüche, **dadurch gekennzeichnet, daß** sie mindestens einen Zusatzstoff enthält, der unter den Konservierungsmitteln, Parfums, Sonnenschutzfiltern, Mitteln gegen freie Radikale, Hydratisierungsmitteln, Vitaminen, Proteinen, Ceramiden und Weichmachern ausgewählt ist.

30. Zusammensetzung nach einem der vorhergehenden Ansprüche, **dadurch gekennzeichnet, daß** die Zusammensetzung wasserfrei ist.

31. Zusammensetzung nach einem der vorhergehenden Ansprüche, **dadurch gekennzeichnet, daß** die Zusammensetzung in Form einer Zusammensetzung zum Schminken oder zur kosmetischen Behandlung der Haut, der Keratinfasern oder der Lippen vorliegt.

32. Verfahren zum Schminken der Haut und/oder der Keratinfasern und/oder der Lippen, **dadurch gekennzeichnet, daß** auf die Haut und/oder die Keratinfasern und/oder die Lippen eine Zusammensetzung nach einem der Ansprüche 1 bis 31 aufgebracht wird.

33. Verfahren zur nicht therapeutischen Behandlung zur Pflege der Haut und/oder der Keratinfasern und/oder der Lippen, **dadurch gekennzeichnet, daß** auf die Haut und/oder die Keratinfasern und/oder die Lippen eine Zusammensetzung nach einem der Ansprüche 1 bis 31 aufgebracht wird.

34. Kosmetische Verwendung einer Zusammensetzung nach einem der Ansprüche 1 bis 31 zur Herstellung eines Films, der sich nicht überträgt (ohne Transfer).

35. Kosmetische Verwendung einer Zusammensetzung nach einem der Ansprüche 1 bis 31 zur Herstellung eines Films, der gegenüber Reibung und/oder Tränen und/oder Schweiß und/oder Sebum hochbeständig ist.

36. Kosmetische Verwendung einer Zusammensetzung nach einem der Ansprüche 1 bis 31 zur Herstellung eines wasserbeständigen Films.

## Claims

1. Cosmetic or dermatological film-forming composition comprising a liquid fatty phase, including at least one volatile hydrocarbon oil, and a film-forming polymer in the said liquid fatty phase, **characterized in that** it comprises at least one polyolefin wax resulting from the homopolymerization of an alpha-olefin corresponding to the general formula: R-CH=CH₂, in which R denotes an alkyl radical having from 10 to 50 carbon atoms, the said wax having a melting point ranging from 50°C to 80°C.

2. Composition according to Claim 1, **characterized in that** the polyolefin wax has a melting point ranging from 50°C to 60°C.

3. Composition according to Claim 1 or 2, **characterized in that** the polyolefin wax has a needle penetrability, measured at 44°C, ranging from 15 to 120.

4. Composition according to any one of the preceding claims, **characterized in that** the polyolefin wax has a needle penetrability, measured at 44°C, ranging from 100 to 120 and better still from 105 to 115.

5. Composition according to any one of the preceding claims, **characterized in that** the said polyolefin wax has a number-average molecular weight ranging from 400 to 3000.

6. Composition according to any one of the preceding claims, **characterized in that** the said polyolefin wax has a number-average molecular weight ranging from 2000 to 3000 and better still from 2500 to 2700.

7. Composition according to any one of the preceding claims, **characterized in that** the said polyolefin wax has a density ranging from 0.85 to 0.95 g/cm³.

8. Composition according to Claim 1, **characterized in that** R denotes an alkyl radical having from 25 to 50 carbon atoms.

9. Composition according to either of Claims 1 and 8, **characterized in that** the alkyl radical R is linear.

10. Composition according to any one of the preceding claims, **characterized in that** the polyolefin wax is present in a content ranging from 0.1% to 10% by weight with respect to the total weight of the composition.

11. Composition according to any one of the preceding claims, **characterized in that** the polyolefin wax is present in a content ranging from 1% to 5% by weight with respect to the total weight of the composition.

12. Composition according to any one of the preceding claims, **characterized in that** the volatile hydrocarbon oil is chosen from the group formed by isoparaffins comprising from 8 to 16 carbon atoms.

13. Composition according to any one of the preceding claims, **characterized in that** the volatile hydrocarbon oil is present in a content ranging from 20% to 99.4% by weight with respect to the total weight of the composition.

14. Cosmetic composition according to any one of the preceding claims, **characterized in that** the volatile hydrocarbon oil is present in a content ranging from 40% to 80% by weight with respect to the total weight of the composition and better still from 50% to 70% by weight.

15. Composition according to any one of the preceding claims, **characterized in that** the liquid fatty phase additionally comprises one or more volatile solvents other than the volatile hydrocarbon oils.

16. Composition according to any one of the preceding claims, **characterized in that** the liquid fatty phase additionally comprises at least one non-volatile oil of mineral, animal, vegetable or synthetic origin.

17. Composition according to any one of the preceding claims, **characterized in that** the film-forming polymer is soluble in the liquid fatty phase.

18. Composition according to Claim 17, **characterized in that** the film-forming polymer is chosen from the copolymers of formula (I) 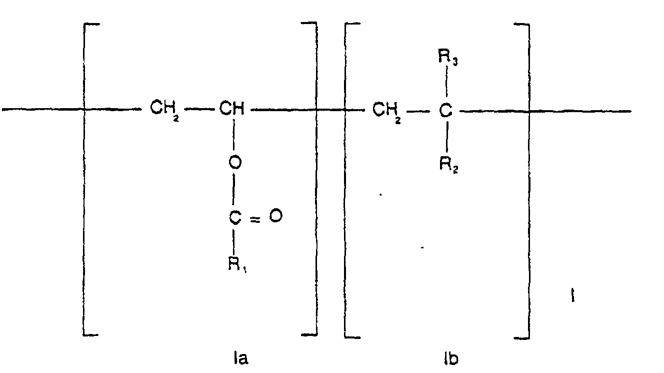 in which:
- R₁ represents a saturated, linear or branched, hydrocarbon chain having from 1 to 19 carbon atoms;
- R₂ represents a radical taken from the group consisting of:
a) -O-CO-R₄, R₄ having the same meaning as R₁ but being
different from R₁ in the same copolymer,
b) -CH₂-R₅, R₅ representing a saturated, linear or branched, hydrocarbon chain having from 5 to 25 carbon atoms,
c) -O-R₆, R₆ representing a saturated hydrocarbon chain having from 2 to 18 carbon atoms, and
d) -CH₂-O-CO-R₇, R₇ representing a saturated, linear or branched, hydrocarbon chain having from 1 to 19 carbon atoms,
- R₃ represents a hydrogen atom when R₂ represents the a), b) or c) radicals or R₃ represents a methyl radical when R₂ represents the d) radical, it being necessary for the said copolymer to be composed of at least 15% by weight of at least one monomer derived from a unit (Ia) or from a unit (Ib) in which the saturated or branched hydrocarbon chains have at least 7 carbon atoms.

19. Composition according to either of Claims 17 and 18, **characterized in that** the film-forming polymer is a homopolymer chosen from the group formed by the homopolymers resulting from the homopolymerization of vinyl esters having from 9 to 22 carbon atoms or alkyl (meth)acrylates where the alkyl radical has from 10 to 20 carbon atoms.

20. Composition according to any one of Claims 17 to 19, **characterized in that** the film-forming polymer is chosen from the group formed by copolymers of C₂-C₂₀ alkenes, alkylcelluloses with a saturated or unsaturated, linear or branched, C₁ to C₈ alkyl radical, and vinylpyrrolidone copolymers.

21. Composition according to any one of Claims 17 to 20, **characterized in that** the film-forming polymer is chosen from copolymers of vinylpyrrolidone and of a C₂ to C₄₀ alkene.

22. Composition according to any one of the preceding claims, **characterized in that** the film-forming polymer is present in a content ranging from 0.5% to 20% by weight with respect to the total weight of the composition and preferably from 1% to 15% by weight.

23. Composition according to any one of the preceding claims, **characterized in that** it moreover comprises one or more additional waxes of animal, vegetable or synthetic origin.

24. Composition according to Claim 23, **characterized in that** the waxes of animal, vegetable or synthetic origin, other than the said polyolefin wax, are present in a content ranging from 0% to 30% by weight with respect to the total weight of the composition and preferably from 1 to 20% by weight.

25. Composition according to any one of the preceding claims, **characterized in that** it additionally comprises a silicone gum.

26. Composition according to any one of the preceding claims, **characterized in that** it comprises at least one colouring material chosen from the group formed by pulverulent compounds and fat-soluble dyes.

27. Composition according to Claim 26, **characterized in that** the pulverulent compounds are chosen from the group formed by pigments, pearlescent agents and fillers.

28. Cosmetic composition according to any one of the preceding claims, **characterized in that** it comprises at least one agent for thickening the liquid fatty phase.

29. Composition according to any one of the preceding claims, **characterized in that** it comprises at least one additive chosen from the group formed by preservatives, fragrances, sunscreen agents, agents for combating free radicals, moisturizing agents, vitamins, proteins, ceramides and plasticizers.

30. Composition according to any one of the preceding claims, **characterized in that** the composition is anhydrous.

31. Composition according to any one of the preceding claims, **characterized in that** the composition is provided in the form of a composition for making up or cosmetically treating the skin, keratinous fibres or lips.

32. Process for making up the skin and/or keratinous fibres and/or lips, **characterized in that** a composition according to any one of Claims 1 to 31 is applied to the skin and/or keratinous fibres and/or lips.

33. Non-therapeutic treatment process for the care of the skin and/or keratinous fibres and/or lips, **characterized in that** a composition according to any one of Claims 1 to 31 is applied to the skin and/or keratinous fibres and/or lips.

34. Cosmetic use of a composition as defined according to any one of Claims 1 to 31 for producing a film having non-transfer properties.

35. Cosmetic use of a composition as defined according to any one of Claims 1 to 31 for producing a film exhibiting good hold with regard to rubbing and/or to tears and/or to perspiration and/or to sebum.

36. Cosmetic use of a composition as defined according to any one of Claims 1 to 31 for producing a water-resistant film.
